# EUROPÄISCHE PATENTSCHRIFT

(11) **EP 2 354 220 B1**
(45) Veröffentlichungstag und Bekanntmachung des Hinweises auf die Patenterteilung: **09.09.2015**
(21) Anmeldenummer: 11450013.5
(22) Anmeldetag: 28.01.2011
(51) Int. Cl.: C12N 5/0775

(54) **Verfahren zur Herstellung einer mesenchymalen Stammzellenpräparation**
Method for manufacturing a mesenchymal stem cell preparation
Procédé de fabrication d'une préparation de cellules souche mésenchymateuses

(30) Priorität: 29.01.2010 AT 1232010
(43) Veröffentlichungstag der Anmeldung: 10.08.2011
(73) Patentinhaber: Heinrich, Karl Georg, 1010 Wien (AT)
(72) Erfinder: Heinrich, Karl Georg, 1010 Wien (AT)
(74) Vertreter: Sonn & Partner Patentanwälte

(56) Entgegenhaltungen:
- WO-A2-03/053346
- WO-A2-03/084468
- WO-A2-2005/042730
- WO-A2-2008/013863
- US-A1- 2008 299 656
- KOTARO YOSHIMURA ET AL: "Cell-Assisted Lipotransfer for Cosmetic Breast Augmentation: Supportive Use of Adipose-Derived Stem/Stromal Cells", AESTHETIC PLASTIC SURGERY, SPRINGER-VERLAG, NE, Bd. 32, Nr. 1, 1. September 2007 (2007-09-01), Seiten 48-55, XP019586869, ISSN: 1432-5241
- LOCKE MICHELLE ET AL: "Human adipose-derived stem cells: isolation, characterization and applications in surgery", ANZ JOURNAL OF SURGERY, BLACKWELL PUBLISHING ASIA, CARLTON SOUTH, AU, Bd. 79, Nr. 4, 1. April 2009 (2009-04-01), Seiten 235-244, XP009148148, ISSN: 1445-1433, DOI: DOI:10.1111/J.1445-2197.2009.04852.X [gefunden am 2009-04-01]
- HALVORSEN Y D ET AL: "Thiazolidinediones and glucocorticoids synergistically induce differentiation of human adipose tissue stromal cells: biochemical, cellular, and molecular analysis", METABOLISM, CLINICAL AND EXPERIMENTAL, W.B. SAUNDERS CO., PHILADELPHIA, PA, US, Bd. 50, Nr. 4, 1. April 2001 (2001-04-01), Seiten 407-413, XP009148136, ISSN: 0026-0495, DOI: DOI:10.1053/META.2001.21690 [gefunden am 2002-05-08]
- KUHBIER JOERN W ET AL: "Isolation, characterization, differentiation, and application of adipose-derived stem cells", ADVANCES IN BIOCHEMICAL ENGINEERING, BIOTECHNOLOGY, Bd. 123, 21. Januar 2010 (2010-01-21), Seiten 55-105, XP009148140, ISSN: 0724-6145, DOI: DOI:10.1007/10_2009_24 [gefunden am 2010-01-21]

## Beschreibung

Die vorliegende Erfindung betrifft ein Verfahren zur Herstellung einer mesenchymalen Stammzellenpräparation.

Das aus dem eigenen Fettdepot gewonnene Fett ist das natürlichste Material, womit das Gesicht modelliert, verjüngt und die Falten aufgefüllt werden können. Die besondere Aufbereitung der Fettzellen sowie geschickte Faltenunterspritzung mit Eigenfett straffen das Gesicht mit und sorgen dabei in den meisten Fällen für eine lang andauernde Haltbarkeit der Behandlung.

Die Vorteile der Eigenfettunterspritzung sind die Allergiefreiheit, Natürlichkeit, keine Fremdkörperreaktion, keine harten Knoten, das Eigenfett steht in großen Mengen zur Verfügung - somit lassen sich größere Gesichtsareale aufpolstern und ohne Messer liften. Die Haltbarkeit kann Jahrzehnte andauern, wenn man die richtige Menge einspritzen lässt. Das Verfahren muss nach heutigen Erkenntnissen mindestens 2-3 mal wiederholt werden, um die langfristige Polsterung und Füllung zu erreichen. Bei einmaliger Gabe bleibt lediglich etwa 10-20% Eigenfett angewachsen im Körper.

In Yoshimura et al. (Aesth Plast Surg, 31, (2008), S. 48-55) werden Verfahren zur Brustvergrößerung geoffenbart, bei denen körpereigenes Fettgewebe verwendet wird. Dabei wird zunächst Körperfett abgesaugt und halbiert. Aus einer Hälfte des Fettgewebes wird die stromavaskuläre Fraktion, welche Stammzellen enthält, gewonnen. Die aus dem Fettgewebe isolierten Zellen werden schließlich zur anderen Hälfte des unbehandelten Fetts hinzugegeben.

In Yoshimura et al. (Dermatologic Surgery, 34 (2008), S. 1178-1185) und Sterodimas et al. (Aesthetic Surgery Journal, 30, (2010), S. 78-81) wird ein vergleichbares Verfahren geoffenbart.

In US 2003/0161816 A1, WO 2008/013863 A2, WO 2005/042730 A2 und WO 2003/084468 A2 werden Verfahren zur Isolierung von Zellen, insbesondere von Stammzellen aus Fettgewebe beschrieben.

Es ist Aufgabe der vorliegenden Erfindung eine Präparation zur Verfügung zu stellen, die es ermöglicht, die Anzahl der kosmetischen Eingriffe so gering wie möglich zu halten (idealerweise ein, maximal zwei Eingriffe) und gleichzeitig das Anwachsen von Eigenfett zu fördern und die Anwachsrate signifikant zu erhöhen.

Die vorliegende Erfindung betrifft ein Verfahren zur Reinigung und Gewinnung einer mesenchymalen Stammzellenpräparation aus Fettgewebe zum Aufspritzen von Gewebe umfassend die Schritte:
a) Trennen des Fettes eines bereitgestellen Fettgewebes von überschüssigem Wasser, Öl und/oder Blut durch Zentrifugation,
b) Teilen des abzentrifugierten Fettes aus Schritt a) und inkubieren von 40% des abzentrifugierten Fettes mit Kollagenase bei 30° bis 40°C, vorzugsweise bei 37°C, für 10 - 45 min, vorzugsweise für 20 bis 40 min,
c) Isolieren von mesenchymalen Stammzellen aus dem verdauten Fett durch Zentrifugation,
d) Vermischen der isolierten Stammzellen aus Schritt c) mit dem unverdauten Fett aus Schritt b), wobei der Anteil an unverdautem Fett 60% beträgt.

Es hat sich überraschender Weise herausgestellt, dass eine Stammzellenpräparation, welche mit diesem Verfahren hergestellt wird, besonders gut geeignet ist, um für kosmetische Zwecke, wie beispielsweise Falten- und Gewebsunterspritzungen, eingesetzt zu werden. Ein entscheidender Schritt im erfindungsgemäßen Verfahren ist, dass ein Teil des einem Patienten entnommenen Fettgewebes unbehandelt, aber mit mesenchymalen Stammzellen angereichert, demselben Patienten rückgeführt wird. D.h. einem Patienten wird zunächst Fettgewebe entnommen, mit dem erfindungsgemäßen Verfahren weiterverarbeitet und schließlich demselben Patienten in Form eine Gewebs- oder Faltenunterspritzung wieder verabreicht.

Durch die Tatsache, dass Fettgewebe mit mesenchymalen Stammzellen angereichert wird, ist es möglich die Anzahl der Eingriffe bei Gewebspolsterungen bzw. -unterspritzungen gering zu halten. Es hat sich gezeigt, dass in der Regel ein einziger Eingriff ausreichend sein kann, um den gewünschten Effekt langfristig zu erzielen. Mit herkömmlichen Verfahren konnte dieser langfristige Effekt nicht erzielt werden, wodurch mehrere, unter Umständen, regelmäßige Eingriffe vonnöten sind. Dieser langfristige Effekt ist vor allem darauf zurückzuführen, dass die erfindungsgemäße Präparation einen erheblichen Anteil an unbehandeltem Fettgewebe aufweist, welches mit mesenchymalen Stammzellen angereichert ist. Dies ermöglicht ein optimales Anwachsen von Fettgewebe am Ort des Eingriffs, so dass mindestens 30%, in der Regel 50 bis 80%, des Fettgewebes am Applikationsort dauerhaft verbleiben. Mit herkömmlichen Verfahren ist dies nicht möglich.

Es hat sich erfindungsgemäß ergeben, dass dieser vorteilhafte Effekt auf das Teilungsverhältnis von verdautem zu unverdautem Fettgewebe zurückzuführen ist. Es hat sich nämlich gezeigt, dass eine Stammzellenpräparation bei der weniger als 30% bzw. mehr als 50% des Ausgangsmaterials verdaut wird, nicht geeignet ist um eine ausgewogene Menge an mesenchymalen Stammzellen bereit zu stellen, welche, vermengt mit der Restmenge an unverdauten Fettgewebe, zu einer Stammzellenpräparation mit den geforderten Eigenschaften führt.

Verfahren zur Bereitstellung von Fettgewebe sind dem Fachmann hinreichend bekannt. Fettgewebe kann beispielsweise durch die Verwendung von Kanülen aus einem Patienten gewonnen werden.

Um die im Fettgewebe enthaltenen mesenchymalen Stammzellen zu isolieren, ist es zu Beginn des Verfahrens von Vorteil, Blutreste, Öl, Wasser usw., das im, z.B., durch Aspiration gewonnenen Fettgewebe vorhanden ist, zu reduzieren bzw. zu entfernen. Dieser Schritt ist zusätzlich von Vorteil, um die Lagerstabilität des Endprodukts zu erhöhen.

Gemäß einer bevorzugten Ausführungsform der vorliegenden Erfindung wird die Zentrifugation in Schritt b) und d) bei 100 x g bis 2000 x g, vorzugsweise 150 x g bis 1000 x g, durchgeführt.

Die Zentrifugationsgeschwindigkeit wird derart gewählt, dass mesenchymale Stammzellen innerhalb der Probe nicht zerstört werden.

Um eine angemessene Verdauung des Fettgewebes zu ermöglichen wird Kollagenase zugesetzt. Die bei der Inkubation gemäß Schritt c) eingesetzte Enzymmenge beträgt vorzugsweise 0,1 bis 10 E/ml, vorzugsweise 0,5 bis 5 E/ml, noch mehr bevorzugt mit 1 bis 3 E/ml, Kollagenase.

Ein weiterer Aspekt der vorliegenden Erfindung betrifft eine Stammzellenpräparation die nach dem erfindungsgemäßen Verfahren erhältlich ist.

Ein noch weiterer Aspekt der vorliegenden Erfindung betrifft die Verwendung einer erfindungsgemäßen Stammzellenpräparation zur Faltenbehandlung, zur Unterspritzung von Gewebe, Regeneration und lokale Verjüngung von Geweben, inklusive Regeneration des Haarwurzelwachstum, sowie generelle, systemische Regenerationsbehandlungen.

Mit der erfindungsgemäßen Präparation lassen sich sehr gut die tiefen Falten, wie z.B Nasolabialfalten anheben, die Lippe vergrößern, Jochbein und Kinn vergrößern und sogar eingesunkene Narben glätten.

Eigenfett ist das bevorzugte Material, wenn in Gewebeschichten Volumen aufgebaut werden soll. Da Fett im Körper aber abgebaut wird, bringt eine Unterspritzung mit Eigenfett oft ein weniger dauerhaftes Ergebnis als die Verwendung anderer Substanzen. Man kann aber davon ausgehen, dass nach ca. sechs Monaten etwa die Hälfte des injizierten Fettes abgebaut ist. Deshalb können zwei bis drei Anschlussbehandlungen erforderlich werden, um den erzielten Effekt auf Dauer konstant zu halten. Durch die Verwendung des erfindungsgemäßen Präparats können diese Nachteile - wie bereits eingangs erwähnt - beseitigt werden.

Aufgrund der hervorragenden Eigenschaften im Hinblick auf die Anwachsrate des Fettgewebes und auf die damit einhergehende Stabilität des Fettgewebes am Applikationsort ist ein kosmetisches Verfahren offenbart, zur Beseitigung und/oder Reduktion von Falten bei einem Patienten umfassend den Schritt des Unterspritzens von Falten mit einer Stammzellenpräparation erhältlich nach einem erfindungsgemäßen Verfahren.

Dieses kosmetische Verfahren umfasst vorzugsweise zusätzlich die folgenden Schritte:
i) Entnehmen von Fettgewebe aus einem Patienten,
ii) Durchführen der Schritte a) bis e) des erfindungsgemäßen Verfahrens zur Gewinnung einer mesenchymalen Stammzellenpräparation aus dem Fettgewebe gemäß i) und
iii) Unterspritzen der Falten des Patienten mit der Stammzellenpräparation gemäß ii).

Die gegenständliche Erfindung wird ferner durch die folgenden Beispiele eingehender illustriert.

### BEISPIELE:

### Beispiel 1:

Zunächst wurde mit 2 mm Absaugkanülen (BD-60cc-Toomeys) das Fettgewebe einer Patientin aus dem Oberschenkelbereich aspiriert und in 50 ml Behälter eingebracht, die zum Zentrifugieren geeignet sind. Anschließend wurde das Fettgewebe in den Behältern bei 3.000 U/min für ca. 6 min zentrifugiert (Hettich-Tisch-Zentrifuge). Dadurch konnte Wasser, Öl und Blut vom Fettgewebe separiert werden.

Das mittels Zentrifugation erhaltene Fettgewebe wurde in verschiedenen Verhältnissen geteilt, wobei ein Verhältnis 6:4 besonders gute Ergebnisse brachte.

Ein Teil des Fettgewebes (ca. 40%) wurde mit Kollagenase (Serva-Collagenase Grad NB 6 in PBS-Lösung) für ca. 20 min bei ca. 37°C unter leichtem Schütteln angedaut. Die eingesetzte Menge vom Enzym belief sich auf 3 IE pro ml anzudauendem Fettgewebes.

Der restliche Teil des Fettgewebes (ca. 60%) wurde in der Zwischenzeit als Matrix vorbereitet.

Zur Isolierung der Gewebsstammzellen wurde das angedaute Fett bei 3.000 U/min für ca. 6 min zentrifugiert. Das bei der Zentrifugation anfallende Pellet, welches die Stammzellen umfasst, wurde in PBS ("Phosphate Buffered Saline"; "Phosphat-gepufferte Salzlösung"-Lösung) resuspendiert.

Um noch vorhandenes Restenzym zu entfernen, wurde das resuspendierte Pellet nochmals bei 3.000 U/min für 6 min zentrifugiert. Das abgeschiedene Pellet umfassend die zu isolierenden Stammzellen wurde wiederum in PBS resuspendiert.

Die resuspendierten Stammzellen wurden anschließend mit dem restlichen Teil des Fettgewebes (Matrix; ca. 60% der Ausgangsmenge an Fettgewebe) vermischt und in diesem verteilt.

Die daraus hergestellte Matrix aus Eigenfett und Stammzellen wurde anschließend zur Brustvergrößerung eingesetzt.

### Beispiel 2:

In einem Anwendetest wurden diverse mesenchymale Stammzellenpräparationen die in unterschiedlicher Weise hergestellt wurden, bei 20 Teilnehmerinnen im Alter von 25 bis 60 Jahren zur Vergrößerung der Brüste eingesetzt. Die eingesetzten Stammzellenpräparationen wurden gemäß Beispiel 1 hergestellt, wobei jener Anteil des Fettgewebes, welcher mit Kollagenase verdaut wurde in 6 Präparationen variiert wurde. Bei Präparation A wurden 20 %, bei Präparation B 30%, bei Präparation C 40%, bei Präparation D 50%, bei Präparation E 60% des von den Patientinnen gewonnenen Fettgewebes mit Kollagenase versetzt und zur Zellgewinnung verwendet. Diese Präparationen wurden aus dem Fettgewebe von je 4 Patientinnen hergestellt und bei derselbigen zur Vergrößerung der Brüste eingesetzt. In den Abständen von 1 Woche, 3 Wochen, 5 Wochen, 3 Monate, wurden digitalisierte Aufnahmen von den vergrößerten Brüsten gemacht und die Ansicht und das Volumen computerunterstützt ausgewertet. Die Ergebnisse sind in der folgenden Tabelle angeführt.

| Zeitpunkt ab Brustvergrößerung | | | | | |
|---|---|---|---|---|---|
| | Präparation | Präparation | Präparation | Präparation | Präparation |
| | A | B | C | D | E |
| 1 Woche(abz. Schwell.) | 100% | 100% | 100% | 100% | 100% |
| 5 Wochen | 85-90% | 98-100% | 99-100% | 100% | 88-91 % |
| 3 Monate | 70-78% | 95-100% | 98-100% | 97-100% | 80-83% |
| 6 Monate | 61-65% | 89-95% | 95-98% | 96-98% | 72-75% |

Interessanterweise ergab sich eine optimale Volumserhaltung bei Vorgehen nach Präparation C, wo also 40% des vorhandenen Fettes zur Zellisolierung verwendet wurden, nicht etwa - wie eigentlich zu erwarten gewesen wäre - bei D und E, wo höhere Anteile des Fettgewebes zur Zellisolierung herangezogen wurden.

### Beispiel 3:

In weiteren Untersuchungen bei einem längeren Beobachtungszeitraum von 12 Monaten und einer Mehrzahl von 50 Patienten stellte sich schlussendlich heraus, dass die besten Ergebnisse in der Volumserhaltung bei jenen Patienten erzielt werden konnten, denen behandeltes Eigenfettgewebe verabreicht wurde, welches mesenchymale Stammzellen aus 30 bis 40% Ausgangsfettgewebe enthielt.

Der Hintergrund dieses überraschenden Ergebnisses ist das subtile Zusammenwirken von Stammzellen, Progenitorzellen, dem als Matrix dienenden Implantationsfett und der optimalen Menge an Zell-und Gewebewachstumsfaktoren (Adipose Tissue Growth Factors, Angiogenese Factors, Connective Tissue Growth Factors u.a.) im Microenvironment, das für den Erfolg beim Volumserhalt wesentlich wichtiger zu sein scheint, als die absolute Menge an reifen oder unreifen Fettzellen bzw. Stammzellen. Es wird weitere zellphysiologische Grundlagenarbeit erforderlich sein, um im Detail zu erklären, weshalb das erfindungsgemäße Verfahren zu deutlich besseren Ergebnissen führt, als bei den bisherigen bekannten Verfahren, in denen ein Teilungsverhältnis von 50:50 verwendet wird.

Um die Ergebnisse zu verdeutlichen wird im folgenden eine vergleichbare Tabelle wie in Beispiel 2 gezeigt. In dieser Versuchsreihe wurden diverse mesenchymale Stammzellenpräparationen die in unterschiedlicher Weise hergestellt wurden, bei 50 Teilnehmerinnen im Alter von 20 bis 60 Jahren zur Vergrößerung der Brüste eingesetzt. Die eingesetzten Stammzellenpräparationen wurden gemäß Beispiel 1 der vorliegenden Patentanmeldung hergestellt, wobei jener Anteil des Fettgewebes, welcher mit Kollagenase verdaut wurde in 5 Präparationen variiert wurde. Bei Präparation A wurden 20 %, bei Präparation B 30%, bei Präparation C 40%, bei Präparation D 50%, bei Präparation E 60% des von den Patientinnen gewonnenen Fettgewebes mit Kollagenase versetzt und zur Zellgewinnung verwendet. Diese Präparationen wurden aus dem Fettgewebe von je 10 Patientinnen hergestellt und bei derselbigen zur Vergrößerung der Brüste eingesetzt.

Da die injizierten Mengen an stammzellangereichertem Eigenfett naturgemäß von Patientin zu Patientin variierten (zwischen 200 ml und 400ml abzüglich Kochsalzlösung in der Matrix) wurde der prozentuelle Volumserhalt relativ zum injizierten Volumen eruiiert. In Abständen von 1 Woche, 5 Wochen, 3, 6 und 12 Monate wurde das Volumen der vergrößerten Brüsten mittels digitalisierter Fotographie und Verdrängungsmessung ermittelt und der prozentuelle Volumserhalt ausgewertet.

| **Zeitpunkt ab BrustvergröBerung** | | | | | |
|---|---|---|---|---|---|
| | **Vol.-erhalt in % Präparation A(20%)** | **Vol.-erhalt in % Präparation B(30%)** | **Vol.-erhalt in % Präparation C(40%)** | **Vol.-erhalt in % Präparation D(50%)** | **Vol.-erhalt in % Präparation E(60%)** |
| 1 Woche(abz. Schwell.) | 100 | 100 | 100 | 100 | 100 |
| 5 Wochen | 82 | 95 | 100 | 95 | 87 |
| 3 Monate | 70 | 86 | 98 | 89 | 75 |
| 6 Monate | 61 | 75 | 96 | 85 | 65 |
| 12 Monate | 50 | 74 | 95 | 75 | 63 |

Diese Daten belegen deutlich, dass mit Präparation C, wie oben beschrieben, die besten Ergebnisse bzgl. Volumserhaltung erzielt werden können.

## Patentansprüche

1. Verfahren zur Reinigung und Gewinnung einer mesenchymalen Stammzellenpräparation aus Fettgewebe zum Aufspritzen von Gewebe umfassend die Schritte:
a) Trennen des Fettes eines bereitgestellen Fettgewebes von überschüssigem Wasser, Öl und/oder Blut durch Zentrifugation,
b) Teilen des abzentrifugierten Fettes aus Schritt a) und inkubieren von 40% des abzentrifugierten Fettes mit Kollagenase, sowie weitere, Bindegewebe verdauende Enzyme, bei 30° bis 45°C, vorzugsweise 30° bis 37°C, für 10 bis 45 min, vorzugsweise für 20 bis 40 min,
c) Isolieren von mesenchymalen Stammzellen aus dem verdauten Fett durch Zentrifugation,
d) Vermischen der isolierten Stammzellen aus Schritt c) mit dem unverdauten Fett aus Schritt b), wobei der Anteil an unverdautem Fett 60% beträgt.

2. Verfahren nach Anspruch 1, **dadurch gekennzeichnet, dass** die Zentrifugation in Schritt a) und c) bei 100 x g bis 2000 x g, vorzugsweise 150 x g bis 1000 x g, durchgeführt wird.

3. Verfahren nach Anspruch 1 oder 2, **dadurch gekennzeichnet, dass** die Inkubation gemäß Schritt b) mit 0,1 bis 10 E/ml, vorzugsweise 0,5 bis 5 E/ml, noch mehr bevorzugt mit 1 bis 3 E/ml, Kollagenase durchgeführt wird.

4. Stammzellenpräparation erhältlich nach einem Verfahren gemäß einem der Ansprüche 1 bis 3.

## Claims

1. A process for the purification and recovery of a mesenchymal stem cell preparation from fatty tissue for filling of tissue by injection comprising the steps of:
a) separating the fat of a provided fatty tissue from an excess of water, oil and/or blood by centrifugation,
b) dividing the centrifuged fat of step a) and incubating 40 % of the centrifuged fat with collagenase, as well as other connective tissue digesting enzymes, at 30 ° to 45 °C, preferably 30 ° to 37 °C, for 10 to 45 min, preferably for 20 to 40 min,
c) isolating mesenchymal stem cells from the digested fat by centrifugation,
d) mixing the isolated stem cells of step c) with the undigested fat of step b), wherein the proportion of undigested fat is 60 %.

2. The process according to claim 1, **characterized in that** the centrifugation of steps a) and c) is performed at 100 x g to 2000 x g, preferably at 150 x g to 1000 x g.

3. The process according to claim 1 or 2, **characterized in that** the incubation of step b) is carried out with 0.1 to 10 U/ml, preferably with 0.5 to 5 U/ml, even more preferably with 1 to 3 U/ml of collagenase.

4. Stem cell preparation obtainably by a process according to any one of claims 1 to 3.

## Revendications

1. Procédé pour la purification et la récupération d'une préparation de cellules souche mésenchymateuses à partir de tissus adipeux pour une pulvérisation des tissus comprenant les étapes de :
a) séparation de la graisse d'un tissu adipeux préparé d'eau, d'huile et/ou de sang en excès par centrifugation,
b) division de la graisse éliminée par centrifugation de l'étape a) et incubation de 40 % de la graisse séparée par centrifugation avec de la collagénase, ainsi que d'autres enzymes digérant des tissus conjonctifs, à de 30° à 45°C, de préférence de 30° à 37°C, pendant de 10 à 45 min, de préférence pendant de 20 à 40 min,
c) isolement de cellules souche mésenchymateuses de la graisse digérée par centrifugation,
d) mélange des cellules souches isolées de l'étape c) avec la graisse non digérée de l'étape b), la part en graisse non digérée étant de 60 %.

2. Procédé selon la revendication 1, **caractérisé en ce que** la centrifugation dans l'étape a) et c) est réalisée à de 100 x g à 2 000 x g, de préférence de 150 x g à 1 000 x g.

3. Procédé selon la revendication 1 ou 2, **caractérisé en ce que** l'incubation est réalisée selon l'étape b) avec de 0,1 à 10 E/ml, de préférence de 0,5 à 5 E/ml, bien mieux encore avec de 1 à 3 E/ml, de collagénase.

4. Préparation de cellules souche obtenue selon un procédé selon l'une quelconque des revendications 1 à 3.
